# EUROPEAN PATENT APPLICATION

(11) **EP 4 410 193 A1**
(43) Date of publication of application: **07.08.2024**
(21) Application number: 22889839.1
(22) Date of filing: 25.10.2022
(51) Int. Cl.: A61B 5/11, A61B 5/00, A61B 5/01, A61B 5/08

(54) **BIOINFORMATION PROCESSING DEVICE, BIOINFORMATION PROCESSING METHOD, AND PROGRAM**

(30) Priority: 04.11.2021 JP 2021180516; 22.12.2021 JP 2021208384
(71) Applicant: OMRON Corporation, Kyoto 600-8530 (JP)
(72) Inventor: OZAWA, Hisashi, Kyoto-shi, Kyoto 600-8530 (JP); YAWATA, Masaki, Kyoto-shi, Kyoto 600-8530 (JP); MURAI, Akito, Kyoto-shi, Kyoto 600-8530 (JP); TANIMOTO, Yudai, Kyoto-shi, Kyoto 600-8530 (JP); MATSUURA, Keiki, Kyoto-shi, Kyoto 600-8530 (JP); OBATA , Junji, Kyoto-shi, Kyoto 600-8530 (JP); IWADE, Ayaka, Kyoto-shi, Kyoto 600-8530 (JP); KAWAKAMI, Riho, Kyoto-shi, Kyoto 600-8530 (JP); SAITO, Keisuke, Kyoto-shi, Kyoto 600-8530 (JP)
(74) Representative: Isarpatent
(86) International application number: PCT/JP2022/039748
(87) International publication number: WO 2023/080019

(57) **Abstract**

A bioinformation processing device includes a signal reception unit configured to receive a signal related to bioinformation reflected from at least one person to be measured, a candidate region identification unit configured to calculate an arrival direction of the signal and/or a distance to the at least one person to be measured from the signal received and identify a candidate region of the at least one person to be measured using the arrival direction and/or the distance calculated, an information generation unit configured to generate bioinformation corresponding to the candidate region of the at least one person to be measured from the signal received, a position information acquisition unit configured to acquire position information of the at least one person to be measured, and a bioinformation association unit configured to associate the at least one person to be measured with the bioinformation generated, based on the position information acquired.

## Description

### TECHNICAL FIELD

The present invention relates to a technique for processing biological information.

### BACKGROUND ART

Techniques for acquiring biological information of a person to be measured using contactless means such as different types of sensors or radars and associating the acquired biological information with the person to be measured are in practical use. For example, Patent Document 1 proposes a technique for identifying a person to be measured from an image generated by an imaging element and associating separately acquired biological information of the person to be measured with the identified person to be measured.

### CITATION LIST

### PATENT LITERATURE

Patent Document 1: JP 2019-152914 A

### SUMMARY OF INVENTION

### TECHNICAL PROBLEM

However, in the related art, the person to be measured is identified on the basis of a feature such as the pattern of bedding used by the person to be measured. Thus, if a plurality of persons to be measured use bedding with the same pattern, there is a possibility that each person to be measured cannot be associated with the biological information with high accuracy. Further, in the related art, the person to be measured is identified by face authentication. However, when biological information of the person to be measured is to be acquired during sleep and the face of the person to be measured is covered or the face moves to a position where an image cannot be captured by an imaging device, for example, there is a possibility that face authentication cannot be normally performed.

The present invention has been made in view of the above circumstances and provides a technique for accurately associating a person to be measured with biological information.

### SOLUTION TO PROBLEM

To achieve the above-mentioned object, the present invention adopts the following configuration.

According to an aspect of the present invention, provided is a biological information processing device including a signal reception unit configured to receive a signal related to biological information reflected from at least one person to be measured, a candidate region identification unit configured to calculate an arrival direction of the signal and/or a distance to the at least one person to be measured from the signal received and identify a candidate region of the at least one person to be measured using the arrival direction and/or the distance calculated, an information generation unit configured to generate biological information corresponding to the candidate region of the at least one person to be measured from the signal received, a position information acquisition unit configured to acquire position information of the at least one person to be measured, and a biological information association unit configured to associate the at least one person to be measured with the biological information generated, on the basis of the position information acquired. Further, according to an aspect of the present invention, provided is a biological information processing device including a signal reception unit configured to receive signals related to biological information reflected from a plurality of persons, a candidate region identification unit configured to calculate an arrival direction of each of the signals and/or a distance to at least one person to be measured of the plurality of persons from the signals received and identify a candidate region of the at least one person to be measured using the arrival direction and/or the distance calculated, an information generation unit configured to generate biological information corresponding to the candidate region of the at least one person to be measured from the signals received, a position information acquisition unit configured to acquire position information of the at least one person to be measured, and a biological information association unit configured to associate the at least one person to be measured with the biological information generated, on the basis of the position information acquired. Accordingly, it is possible to accurately associate a person to be measured with biological information contactlessly acquired for the person to be measured by an acquisition unit of biological information.

Further, the position information of the at least one person to be measured may be position information acquired by optically measuring an outline of the at least one person to be measured. Further, the position information of the at least one person to be measured may be position information acquired by measuring a distribution of pressure applied to different positions by the at least one person to be measured. Further, the position information of the at least one person to be measured may be position information acquired by measuring a distribution of body temperature of the at least one person to be measured. Further, the position information of the at least one person to be measured may be position information acquired by measuring a distribution of space occupied by the at least one person to be measured. Further, the position information of the at least one person to be measured may be position information acquired by measuring a sound emitted by the at least one person to be measured and a generation location of the sound. Further, the position information of the at least one person to be measured may be position information indicating a space occupied by the at least one person to be measured during measurement. Further, the position information of the at least one person to be measured may be position information specified by a detected position of a tag worn by the at least one person to be measured and including identification information of the at least one person to be measured. Accordingly, on the basis of the various position information described above, it is possible to accurately identify a correspondence relationship between the person to be measured and the biological information using a feature of the biological information of the person to be measured.

Further, the biological information association unit may associate the at least one person to be measured with the biological information generated, on the basis of information identifying the at least one person to be measured acquired in advance. Accordingly, the accuracy of the association of the person to be measured with the biological information can be expected to improve. Further, the biological information processing device described above may further include an output unit configured to output, in a case where a spatial distribution of reflection points of the signal received is abnormal, a notification indicating that the at least one person to be measured and the biological information are not normally associated with each other. Accordingly, it is possible to accurately associate the biological information and the person to be measured while issuing notifications of abnormalities that may hinder the acquisition of the biological information of the person to be measured and thus suppressing such a possibility.

Further, according to an aspect of the present invention, provided is a biological information processing device including a signal reception unit configured to receive signals indicating pressure applied to different positions by at least one person to be measured, a candidate region identification unit configured to identify a candidate region of the at least one person to be measured, on the basis of a distribution of the pressure acquired from the signals received, an information generation unit configured to generate biological information corresponding to the candidate region of the at least one person to be measured from the signals received, a position information acquisition unit configured to acquire position information of the at least one person to be measured, and a biological information association unit configured to associate the at least one person to be measured with the biological information generated, on the basis of the position information acquired. Accordingly, it is possible designate as a target a person to be measured on a bed in which pressure measurement elements are arranged in a planar manner, for example, and accurately associate biological information and the person to be measured.

Further, according to an aspect of the present invention, provided is a biological information processing device including a signal reception unit configured to receive a signal related to biological information reflected from at least one person to be measured, an estimation unit configured to estimate, by machine learning, a number of the persons to be measured from the signal received for, in terms of an arrival direction of the signal and/or a distance to the at least one persons to be measured, each arrival direction and/or distance of a predetermined unit, an information generation unit configured to generate biological information of the at least one person to be measured from the signal received, a classification unit configured to classify the biological information generated for the at least one person to be measured, on the basis of the estimated number of the persons to be measured, a position information acquisition unit configured to acquire position information of the at least one person to be measured, and a biological information association unit configured to associate the at least one person to be measured with the biological information classified, on the basis of the position information acquired. In a case where information related to the number of the persons to be measured is acquired, the classification unit classifies the biological information generated for the at least one person to be measured, on the basis of the information acquired instead of the estimated number of the persons to be measured. Further, according to an aspect of the present invention, provided is a biological information processing device including a signal reception unit configured to receive a signal related to biological information reflected from at least one person to be measured, an information generation unit configured to generate biological information of the at least one person to be measured from the signal received, an acquisition unit configured to acquire information related to a number of the persons to be measured, a classification unit configured to classify the biological information generated for the at least one person to be measured, on the basis of the acquired information related to the number of the persons to be measured, a position information acquisition unit configured to acquire position information of the at least one person to be measured, and a biological information association unit configured to associate the at least one person to be measured with the biological information classified, on the basis of the position information acquired. Accordingly, it is possible to classify biological information using information having higher accuracy than the information of the number of persons to be measured identified by inference.

Note that the present invention can also be regarded as a biological information processing method including at least a portion of the processing described above, a program for causing a computer to execute such a method, and a computer-readable storage medium that stores such a program in a non-transitory manner. The configurations and processing described above can be combined to configure the present invention as long as no technical contradiction arises.

### ADVANTAGEOUS EFFECTS OF INVENTION

According to the present invention, it is possible to accurately associate a person to be measured with biological information acquired from the person to be measured.

### BRIEF DESCRIPTION OF DRAWINGS

FIG. 1 is a diagram schematically illustrating a configuration example of a biological information processing device to which the present invention is applied.
FIG. 2 is a diagram illustrating a schematic configuration of the biological information processing device according to an embodiment.
FIG. 3 is a block diagram illustrating an example of the biological information processing device according to an embodiment.
FIG. 4 is a flowchart illustrating an example of a processing flow of the biological information processing device according to an embodiment.
FIG. 5A is a graph showing an example of candidate regions of persons to be measured in an embodiment, FIG. 5B is a graph showing an example of position information of persons to be measured in an embodiment, and FIG. 5C to FIG. 5E are graphs showing examples of biological information of the persons to be measured in an embodiment.
FIG. 6A is a diagram schematically illustrating a configuration example of the biological information processing device according to an embodiment, and FIG. 6B is a graph showing an example of candidate regions of persons to be measured according to an embodiment.
FIG. 7A is a diagram schematically illustrating a configuration example of the biological information processing device according to an embodiment, and FIG. 7B is a graph showing an example of candidate regions of persons to be measured according to an embodiment.
FIG. 8A is a diagram schematically illustrating a configuration example of the biological information processing device according to an embodiment, and FIG. 8B is a graph showing an example of candidate regions of persons to be measured according to an embodiment.
FIG. 9A is a diagram schematically illustrating a configuration example of the biological information processing device according to an embodiment, and FIG. 9B is a diagram illustrating an example of candidate regions of persons to be measured according to an embodiment.
FIG. 10A is a diagram schematically illustrating a configuration example of the biological information processing device according to an embodiment, and FIG. 10B is a diagram illustrating an example of candidate regions of persons to be measured according to an embodiment.
FIG. 11A is a diagram schematically illustrating a configuration example of the biological information processing device according to a modified example, and FIG. 11B is a diagram schematically illustrating a configuration example of a bed in a modified example.
FIG. 12A is a flowchart illustrating an example of a processing flow of the biological information processing device according to a modified example, and FIG. 12B is a graph showing an example of candidate regions of persons to be measured in a modified example.
FIG. 13 is a flowchart illustrating an example of a processing flow of the biological information processing device according to a modified example.
FIG. 14 is a flowchart illustrating an example of a processing flow of the biological information processing device according to a modified example.

### DESCRIPTION OF EMBODIMENTS

### <Application Example>

An application example of the present invention will be described. In the related art, a person to be measured is identified on the basis of an image of the person to be measured. If a plurality of persons to be measured use the same bedding, for example, since there is little difference between the images of the persons to be measured, it may not be possible to identify each person to be measured with high accuracy. Further, in the related art, depending on the position of the person to be measured, there is a possibility that the person to be measured cannot be correctly captured by the imaging device and face authentication cannot be normally performed.

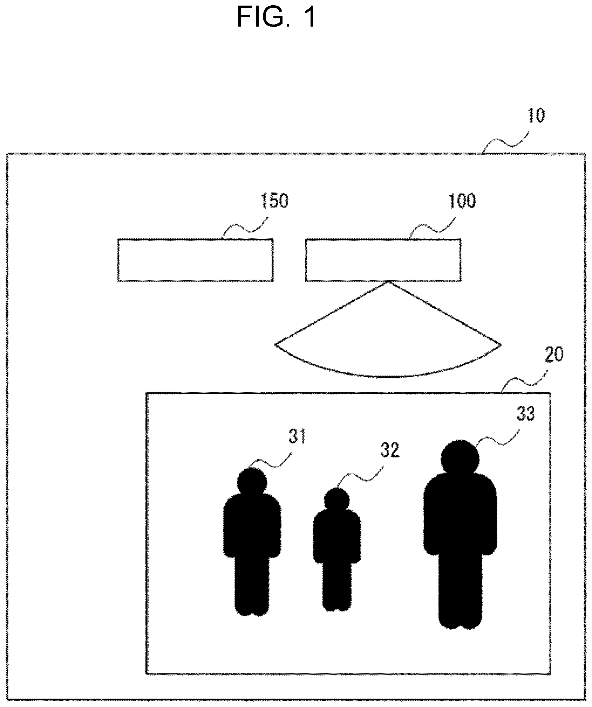

FIG. 1 is a diagram schematically illustrating a usage example of a biological information processing device 100 to which the present invention is applied. In the usage example illustrated in FIG. 1, three persons 31, 32, 33, whose biological information is to be measured, are lying side by side on a bed 20 placed in a room 10. The biological information processing device 100 and a light detection and ranging (LiDAR) scanner 150 are disposed in the room 10. The biological information processing device 100 transmits a signal to the persons 31, 32, 33 to be measured on the bed 20 to perform so-called contactless biological information sensing. Examples of frequencies of the signal transmitted to the persons 31, 32, 33 to be measured include frequencies in the band of 30 GHz to 300 GHz used in a millimeter-wave radar. However, frequencies in other bands such as those of light, radio waves, sound waves, and ultrasonic waves may be employed. The LiDAR scanner 150 is a device that measures the position of the person to be measured by LiDAR technology.

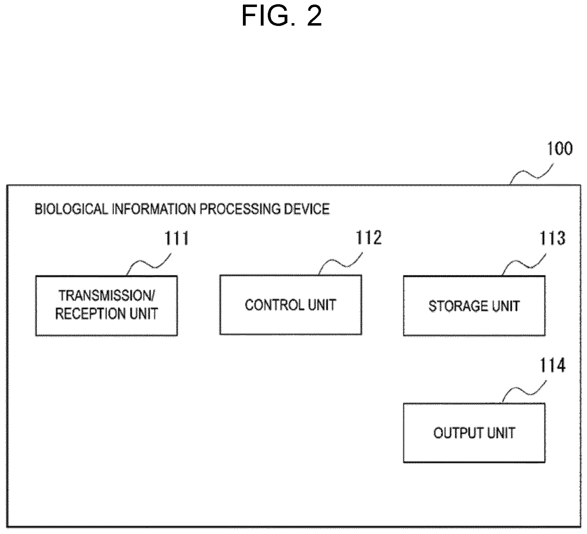

FIG. 2 is a diagram illustrating a configuration example of the biological information processing device 100. The biological information processing device 100 includes a transmission/reception unit 111, a control unit 112, a storage unit 113, and an output unit 114. The transmission/reception unit 111 functions as a signal reception unit that receives a signal related to biological information reflected from a person to be measured, and transmits and receives signals to and from the persons 31, 32, 33 to be measured. The control unit 112 generates biological information of each person to be measured using the signals received by the transmission/reception unit 111 from the persons 31, 32, 33 to be measured. Details of biological information generation processing executed by the control unit 112 will be described below. The storage unit 113 stores various types of data such as signal data received by the transmission/reception unit 111 and data used or generated in processing executed by the control unit 112. The output unit 114 notifies a user of a result of the processing executed by the control unit 112, or outputs data related to the result to an external device. Note that the output unit 114 may be configured to output data related to the biological information of the person to be measured to an external device by various communication methods, such as various wireless communication methods and wired communication methods.

The biological information processing device 100 performs contactless biological information sensing on a plurality of persons to be measured by using a signal transmission/reception unit such as a radio wave radar, an ultrasonic sensor, or a sound wave sensor, and identifies the acquired biological information of each person to be measured on the basis of position information of the persons to be measured. Therefore, according to the biological information processing device 100, it is possible to accurately associate the biological information acquired from the persons to be measured with the persons to be measured.

### <Description of Embodiments>

An embodiment of the technique of the present disclosure will be described. In the present embodiment, as an example, a case is assumed in which the biological information processing device 100 and the bed 20 are arranged in the room 10 as illustrated in FIG. 1, the plurality of persons 31, 32, 33 whose biological information is to be measured lie on the bed 20, and biological information related to respiration of each person to be measured during sleep is acquired by the biological information processing device. Here, it is assumed that the persons 31, 32, 33 to be measured constitute one family and are a woman, a child, and a man, respectively. Note that the biological information to be acquired is assumed to be biological information related to the respiration of the persons to be measured, but the biological information to be acquired may be biological information related to heart rate, body motion, or the like.

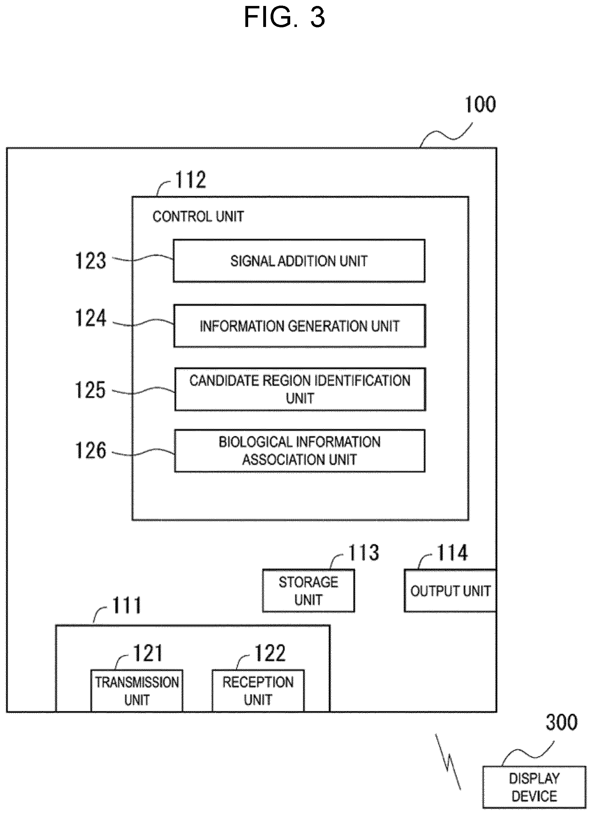

FIG. 3 is a block diagram illustrating a configuration example of the biological information processing device 100 according to an embodiment. As illustrated in FIG. 3, in the biological information processing device 100, the transmission/reception unit 111 includes a transmission unit 121 that transmits a signal to the person to be measured and a reception unit 122 that receives a signal reflected from the person to be measured. Furthermore, the transmission/reception unit 111 communicates with the LiDAR scanner 150 to acquire position information of each person to be measured that was measured by the LiDAR scanner 150. The LiDAR scanner 150 is an example of a device that generates position information of a person to be measured by optically measuring an outline of the person to be measured. Thus, the position information of each person to be measured can be appropriately generated even when the persons to be measured cannot be correctly identified by known image recognition processing of a person to be measured due to inappropriate posture of the person to be measured or the persons to be measured having the same bedding.

Further, the control unit 112 includes a signal addition unit 123 that performs signal addition by adding the signals received by the reception unit 122, an information generation unit 124 that performs signal processing on the signal obtained by addition to generate biological information, a candidate region identification unit 125 that identifies a candidate region indicating a location of a person to be measured, and a biological information association unit 126 that associates biological information with each person to be measured. The biological information of each person to be measured associated by the biological information association unit 126 is output to a display device 300 by the output unit 114. Examples of the display device include a display and an information processing terminal (e.g., a smartphone).

The control unit 112 includes a central processing unit (CPU), a random access memory (RAM), and a read only memory (ROM), and controls each unit in the biological information processing device 100, performs various kinds of information processing, and the like. Further, the storage unit 113 stores a program executed by the control unit 112, and various data used in processing executed by the control unit 112. For example, the storage unit 113 is an auxiliary storage device such as a hard disk drive or a solid state drive. The output unit 114 outputs, to the display device 300, the biological information of the person to be measured generated by the processing of the control unit 112. Note that the biological information generated by the control unit 112 may be stored in the storage unit 113 and output from the output unit 114 to the display device 300 at a desired timing.

Although the biological information processing device 100 and the display device 300 are separate devices in the present embodiment, the biological information processing device 100 may be integrated with the display device 300. Further, at least some of the functions of the biological information processing device 100 may be implemented by a computer on a cloud, or may be a microcomputer such as a programmable logic controller (PLC) or a single board computer.

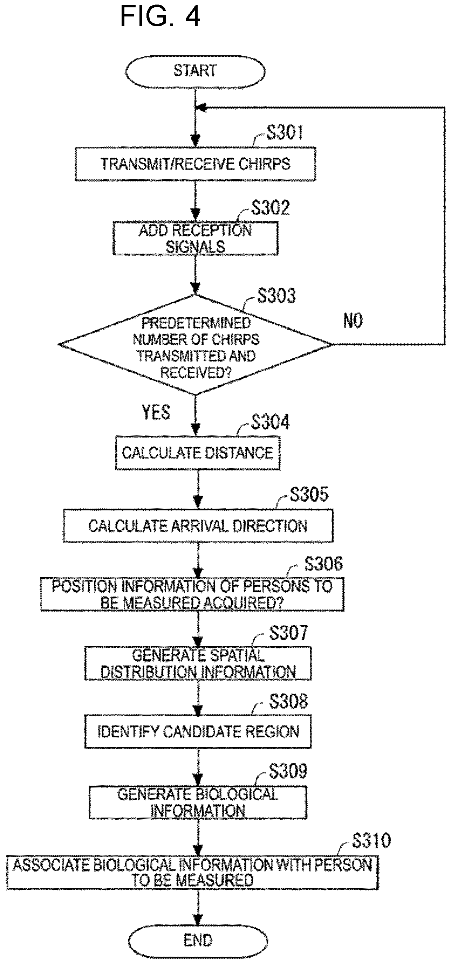

FIG. 4 is a flowchart illustrating an example of a processing flow of the biological information processing device 100. As an example, the processing of FIG. 4 is executed when a user operates the biological information processing device 100 after power-on and instructs the start of the processing flow of FIG. 4. Before the processing of this flowchart is started, information indicating that the persons whose biological information is to be acquired are the persons 31, 32, 33 to be measured, and feature information related to a feature of the biological information of the persons 31, 32, 33 to be measured are stored in advance in the storage unit 113. Alternatively, the user of the biological information processing device 100 may specify this information before the processing of the flowchart is started. Note that the feature information of the biological information of each person to be measured is information used to associate the biological information generated by processing (described below) by the biological information processing device 100 with each person to be measured. In the present embodiment, as an example, the feature information is information indicating a feature of the outline of the body of each person to be measured. The processing for identifying the biological information of each person to be measured, which is executed by the biological information processing device 100, will now be described with reference to FIG. 4.

In step S301, the transmission unit 121 transmits a chirp to the persons 31, 32, 33, whose biological information is to be measured, on the bed 20. Note that the frequency band of the chirp transmitted by the transmission unit 121 and the transmission method, such as up-chirp or down-chirp, may be set as appropriate. Here, as an example, a frequency modulated continuous wave (FMCW) method, a transmission/reception sampling cycle of about 100 µs, and an antenna array with eight channels are assumed. Additionally, the reception unit 122 receives signals reflected from the persons 31, 32, 33 to be measured. Next, in step S302, the signal addition unit 123 performs signal addition by adding intermediate frequency (IF) signals obtained from a difference between the chirp transmitted by the transmission unit 121 and the signal received by the reception unit 122.

In step S303, the control unit 112 determines whether the transmission and reception of chirps to and from the persons 31, 32, 33 to be measured in step S301 was executed a predetermined number of times. Performing the transmission and reception of the chirp a predetermined number of times makes it possible to ensure the accuracy of the biological information generated by the processing described below for the person to be measured. The control unit 112 advances the processing to step S304 in a case where the transmission and reception of the chirp was executed the predetermined number of times (S303: YES), and returns the processing to step S301 in a case where the number of executions of the transmission and reception of the chirp does not satisfy the predetermined number of times (S303: NO). Note that the predetermined number of times of the transmission and reception of a chirp may be determined as appropriate in accordance with the type of biological information to be generated or other factors.

In step S304, the information generation unit 124 calculates a distance from the transmission/reception unit 111 (biological information processing device 100) by using the signal obtained by addition in step S302. Specifically, the information generation unit 124 calculates the distance to the position where the signal is reflected from a different frequency spectrum obtained by performing Fast Fourier transform (FFT) after analog-to-digital (AD) conversion of the IF signals added in step S302.

In step S305, the information generation unit 124 calculates a direction with respect to the transmission/reception unit 111 (the biological information processing device 100) using the signal obtained by addition in step S302. Specifically, the information generation unit 124 calculates the arrival direction (angle) from a difference in phases of reception signals between the plurality of antennas of the reception unit 122.

Next, in step S306, the transmission/reception unit 111 acquires the position information of the persons 31, 32, 33 to be measured that was measured by the LiDAR scanner 150. Next, in step S307, the information generation unit 124 generates information related to a spatial distribution of reflection points received by the reception unit 122 on the basis of each distance and direction calculated in steps S304 and S305. Then, in step S308, the information generation unit 124 identifies a candidate region of the person to be measured on the basis of the information related to the spatial distribution of the reflection points.

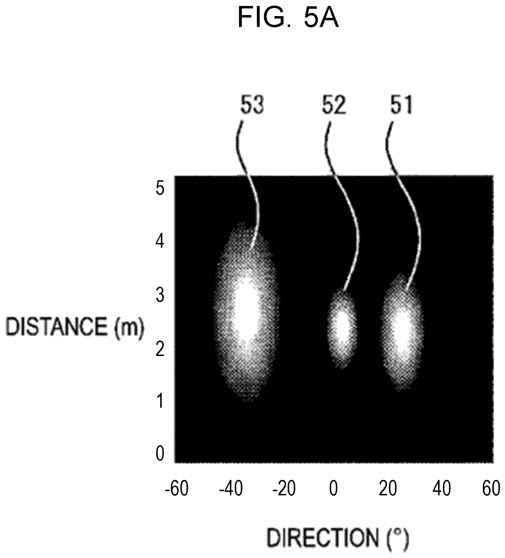

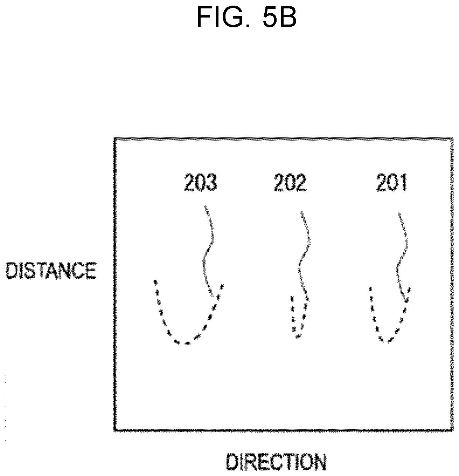

FIG. 5A shows an example of candidate regions identified by the information generation unit 124 on the basis of the information related to the spatial distribution of the reflection points in the present embodiment. Note that regions 51, 52, 53 correspond to the persons 31, 32, 33 to be measured. Further, FIG. 5B shows an example of the position information of the persons 31, 32, 33 to be measured that is received by the transmission/reception unit 111 from the LiDAR scanner 150 in the present embodiment. As shown in FIG. 5B, point clouds 201, 202, 203 indicating the outlines of the persons 31, 32, 33 to be measured are output as position information from the LiDAR scanner 150. As shown in the graph, the point cloud 201 (size: medium), the point cloud 202 (size: small), and the point cloud 203 (size: large) corresponding to the sizes of the persons 31, 32, and 33 to be measured, respectively, are acquired.

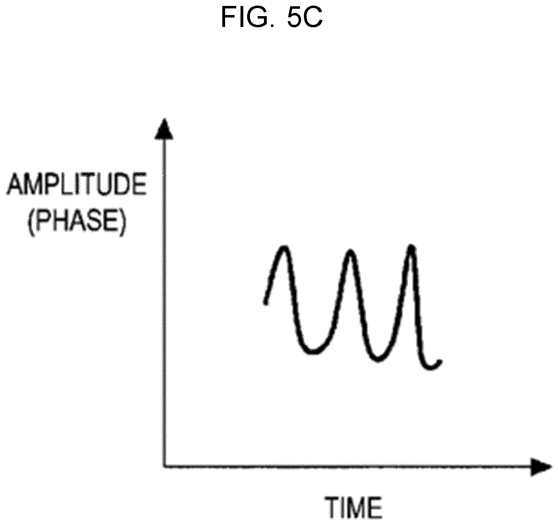

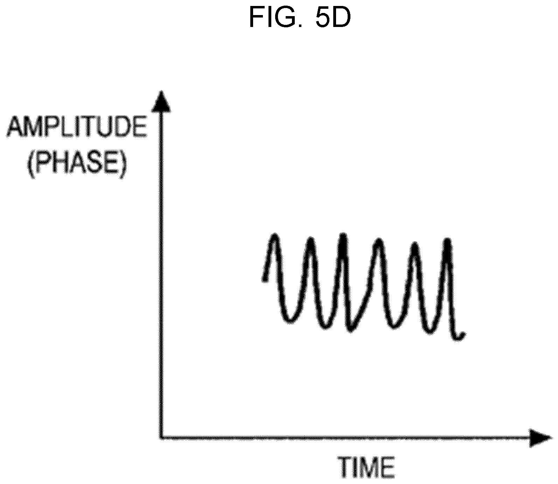

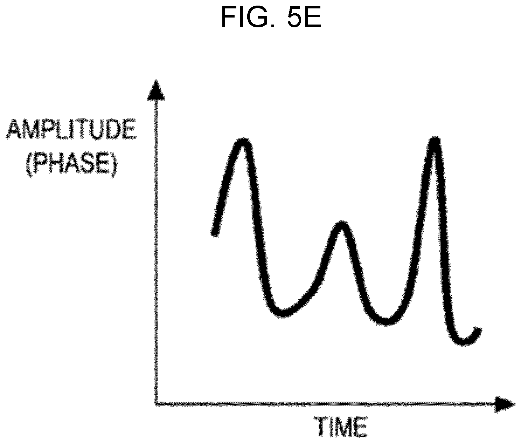

Next, in step S309, for each of the candidate regions 51, 52, 53 identified in step S308, the information generation unit 124 generates biological information indicating a respiratory waveform from a temporal change in amplitude or phase by using the signal obtained by addition in step S302. FIG. 5C, FIG. 5D, and FIG. 5E are examples of graphs showing temporal changes (respiratory waveforms) in amplitude or phase in the candidate regions 51, 52, 53 in the present embodiment. As shown in FIG. 5C, FIG. 5D, and FIG. 5E, biological information indicating respiratory waveforms having different temporal changes in amplitude or phase is acquired for each candidate region 51, 52, 53.

Then, in step S310, the biological information association unit 126 associates the persons 31, 32, 33 to be measured with the biological information on the basis of a degree of similarity between a feature of the outlines of the bodies of the persons 31, 32, 33 to be measured indicated by the position information of the persons 31, 32, 33 to be measured that was measured by the LiDAR scanner 150 and acquired in step S306 and a feature related to the outlines of the bodies of the persons 31, 32, 33 to be measured stored in the storage unit 113. For example, the biological information association unit 126 determines that the point cloud 201 is the outline of the person 31 to be measured from the feature (size: medium) of the outline indicated by the point cloud 201, and associates the biological information acquired from the candidate region 51 corresponding to the position of the point cloud 201 with the person 31 to be measured. Similarly, the biological information association unit 126 associates the biological information acquired from the candidate regions 52, 53 with the persons 32, 33 to be measured, respectively.

According to the present embodiment, on the basis of position information of each person to be measured acquired by optically measuring the outline of each person to be measured by a LiDAR scanner, it is possible to accurately associate the biological information acquired for a plurality of persons to be measured by a biological information acquisition unit using a contactless method, such as a radar, with each person to be measured.

### (Second Embodiment)

Next, the biological information processing device according to a second embodiment will be described. Note that, in the following description, the same configurations, processing, and the like as those of the first embodiment are denoted by the same reference signs, and detailed description thereof will be omitted.

In the biological information processing device 100 according to the first embodiment, each of the candidate regions 51, 52, 53 and each of the persons 31, 32, 33 to be measured are associated with each other on the basis of the position information of each person to be measured acquired by optically measuring the outline of each person whose biological information is to be measured by a LiDAR scanner. However, in the biological information processing device 100 according to the second embodiment, the candidate regions 51, 52, 53 and the persons 31, 32, 33 to be measured are associated with each other on the basis of feature information related to a pressure distribution of each person whose biological information is to be measured on a body pressure sensor. Further, in the present embodiment, before the processing of the flowchart illustrated in FIG. 4 is started, information indicating that the persons whose biological information is to be acquired are the persons 31, 32, 33 to be measured and information related to the feature (e.g., pressure distribution) related to the biological information of each person whose biological information is to be measured acquired from the body pressure sensor are stored in advance in the storage unit 113. Alternatively, the user of the biological information processing device 100 may specify this information before the processing of the flowchart is started.

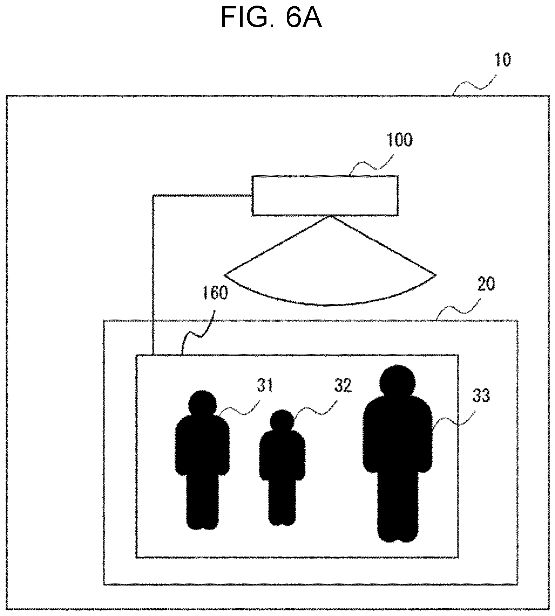

FIG. 6A illustrates a usage example of the biological information processing device 100 according to the present embodiment. As illustrated in FIG. 6A, a body pressure sensor 160 is disposed on the bed 20, and the persons 31, 32, 33 to be measured lie on the body pressure sensor 160. The body pressure sensor 160 measures a distribution of pressure applied to different positions by each person to be measured. Then, feature information indicating the pressure distribution on the body pressure sensor 160 for each of the persons 31, 32, 33 to be measured is output to the biological information processing device 100. The transmission/reception unit 111 acquires the feature information of the pressure distribution of each person to be measured that was measured by the body pressure sensor 160.

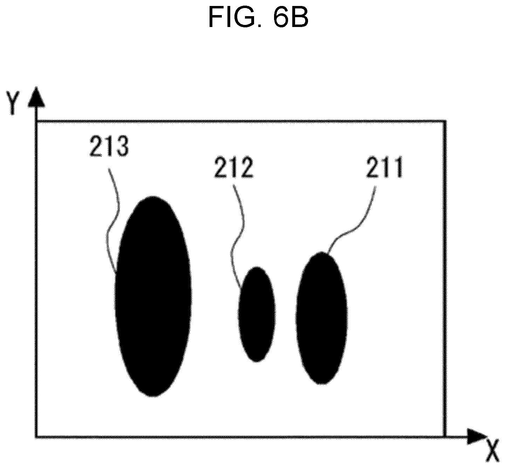

In the present embodiment, in step S306, the transmission/reception unit 111 acquires the feature information indicating the pressure distribution of the persons 31, 32, 33 to be measured that was measured by the body pressure sensor 160. FIG. 6B shows an example of the feature information of the pressure distribution of the persons 31, 32, 33 to be measured that is received by the transmission/reception unit 111 from the body pressure sensor 160 in the present embodiment. As shown in FIG. 6B, a region 211 (size: medium), a region 212 (size: small), and a region 213 (size: large) indicating the pressure distribution on the body pressure sensor 160 according to the body sizes of the persons 31, 32, 33 to be measured are output from the body pressure sensor 160 as position information.

Then, in step S310, the biological information association unit 126 associates the persons 31, 32, 33 to be measured with the biological information on the basis of a degree of similarity between the features of the pressure distributions of the persons 31, 32, 33 to be measured output from the body pressure sensor 160 and acquired in step S306 and the features of the pressure distributions of the persons 31, 32, 33 to be measured stored in the storage unit 113. For example, the biological information association unit 126 determines that the region 211 is the pressure distribution of the person 31 to be measured from the feature (size: medium) of the region 211 indicating the pressure distribution, and associates the biological information acquired from the candidate region 51 corresponding to the position of the region 211 with the person 31 to be measured. Similarly, the biological information association unit 126 associates the biological information acquired from the candidate regions 52, 53 with the persons 32, 33 to be measured, respectively.

Therefore, according to the present embodiment, on the basis of the position information of the pressure distribution of each person to be measured acquired by the body pressure sensor, it is possible to accurately associate the biological information acquired for a plurality of persons to be measured by a biological information acquisition unit using a contactless method, such as a radar, with each person to be measured. Note that, instead of or in addition to the above, the transmission/reception unit 111 may acquire information indicating a weight of each person to be measured from the body pressure sensor 160, and the biological information association unit 126 may associate the biological information with each person to be measured on the basis of the feature information of the pressure distribution of each person to be measured and/or the information indicating the weight of each person to be measured.

### (Third Embodiment)

Next, the biological information processing device according to a third embodiment will be described. Note that, in the following description, the same configurations, processing, and the like as those of the embodiments described above are denoted by the same reference signs, and detailed description thereof will be omitted.

In the biological information processing device 100 according to the first embodiment, each of the candidate regions 51, 52, 53 and each of the persons 31, 32, 33 to be measured are associated with each other on the basis of the position information of each person to be measured acquired by optically measuring the outline of each person whose biological information is to be measured by a LiDAR scanner. However, in the biological information processing device 100 according to the third embodiment, the candidate regions 51, 52, 53 and the persons 31, 32, 33 to be measured are associated with each other on the basis of feature information of a body temperature distribution of each person whose biological information is to be measured, the body temperature distribution being measured by a thermal imaging sensor. Further, in the present embodiment, before the processing of the flowchart illustrated in FIG. 4 is started, information indicating that the persons whose biological information is to be acquired are the persons 31, 32, 33 to be measured and information related to the feature (e.g., body temperature distribution) related to the biological information of each person to be measured that is acquired from the thermal imaging sensor are stored in advance in the storage unit 113. Alternatively, the user of the biological information processing device 100 may specify this information before the processing of the flowchart is started.

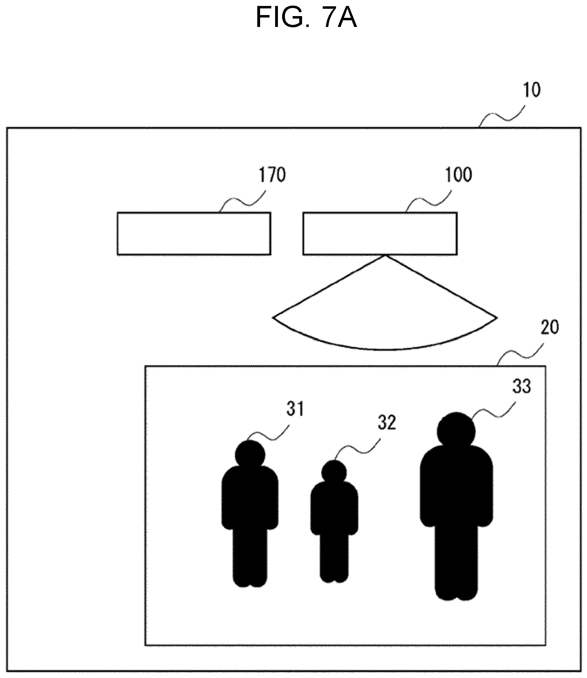

FIG. 7A illustrates a usage example of the biological information processing device 100 according to the present embodiment. As illustrated in FIG. 7A, a thermal imaging sensor 170 is disposed in the room 10. The thermal imaging sensor 170 measures the body temperature of each of the persons 31, 32, 33 to be measured, and outputs feature information indicating a body temperature distribution for each of the persons 31, 32, 33 to be measured to the biological information processing device 100. The transmission/reception unit 111 acquires the feature information of the body temperature distribution of each person to be measured that was measured by the thermal imaging sensor 170.

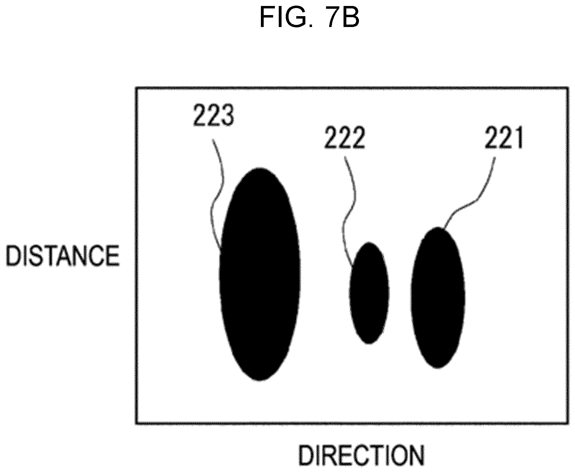

In the present embodiment, in step S306, the transmission/reception unit 111 acquires the feature information indicating the body temperature distribution of the persons 31, 32, 33 to be measured that was measured by the thermal imaging sensor 170. FIG. 7B shows an example of the feature information of the body temperature distribution of the persons 31, 32, 33 to be measured, which is received by the transmission/reception unit 111 from the thermal imaging sensor 170 in the present embodiment. As shown in FIG. 7B, the region 221 (size: medium), the region 222 (size: small), and the region 223 (size: large) indicating the body temperature distribution according to the body sizes of the persons 31, 32, 33 to be measured are output from the thermal imaging sensor 170 as position information.

Then, in step S310, the biological information association unit 126 associates the persons 31, 32, 33 to be measured with the biological information on the basis of a degree of similarity between the features of the body temperature distributions of the persons 31, 32, 33 to be measured output from the thermal imaging sensor 170 and acquired in step S306 and the features of the body temperature distributions of the persons 31, 32, 33 to be measured stored in the storage unit 113. For example, the biological information association unit 126 determines that the region 221 is the body temperature distribution of the person 31 to be measured from the feature (size: medium) of the region 221 indicating the body temperature distribution, and associates the biological information acquired from the candidate region 51 corresponding to the position of the region 221 with the person 31 to be measured. Similarly, the biological information association unit 126 associates the biological information acquired from the candidate regions 52, 53 with the persons 32, 33 to be measured, respectively.

Therefore, according to the present embodiment, on the basis of the position information of the body temperature distribution of each person to be measured acquired by the thermal imaging sensor, it is possible to accurately associate the biological information acquired for a plurality of persons to be measured by a biological information acquisition unit using a contactless method, such as a radar, with each person to be measured. Note that, instead of or in addition to the above, the transmission/reception unit 111 may acquire information indicating the body temperature of each person to be measured from the thermal imaging sensor 170, and the biological information association unit 126 may associate the biological information with each person to be measured on the basis of the feature information of the body temperature distribution of each person to be measured and/or the information indicating the body temperature of each person to be measured.

### (Fourth Embodiment)

Next, the biological information processing device according to a fourth embodiment will be described. Note that, in the following description, the same configurations, processing, and the like as those of the embodiments described above are denoted by the same reference signs, and detailed description thereof will be omitted.

In the biological information processing device 100 according to the first embodiment, each of the candidate regions 51, 52, 53 and each of the persons 31, 32, 33 to be measured are associated with each other on the basis of the position information of each person to be measured acquired by optically measuring the outline of each person whose biological information is to be measured by a LiDAR scanner. However, in the biological information processing device 100 according to the fourth embodiment, the candidate regions 51, 52, 53 and the persons 31, 32, 33 to be measured are associated with each other on the basis of feature information of a spatial distribution occupied by each person whose biological information is to be measured, the spatial distribution being measured by a rotating type radar for spatial distribution detection, for example. Further, in the present embodiment, before the processing of the flowchart illustrated in FIG. 4 is started, information indicating that the persons whose biological information is to be acquired are the persons 31, 32, 33 to be measured and information related to the feature (e.g., size of spatial distribution occupied by the person to be measured) related to the biological information of each person to be measured acquired from the radar for spatial distribution detection are stored in advance in the storage unit 113. Alternatively, the user of the biological information processing device 100 may specify this information before the processing of the flowchart is started.

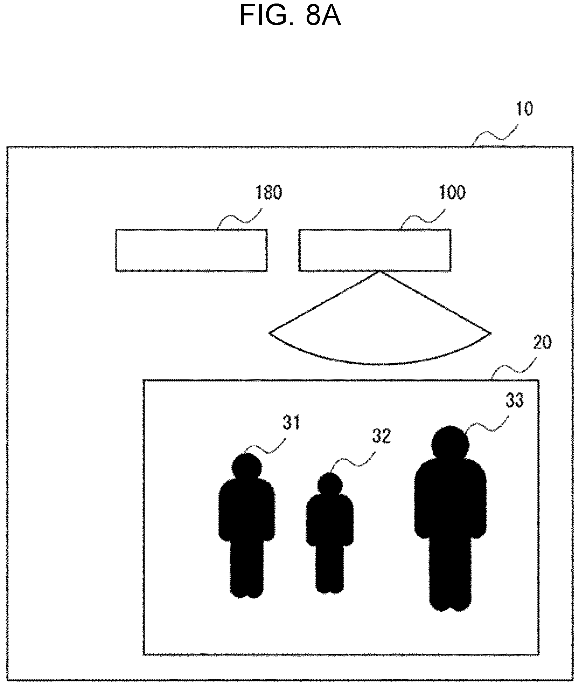

FIG. 8A illustrates a usage example of the biological information processing device 100 according to the present embodiment. As illustrated in FIG. 8A, a radar 180 for spatial distribution detection is disposed in the room 10. The radar 180 measures a spatial distribution occupied by each of the persons 31, 32, 33 to be measured, and outputs feature information indicating the spatial distribution of each of the persons 31, 32, 33 to be measured to the biological information processing device 100. The transmission/reception unit 111 acquires the feature information of the spatial distribution occupied by each person to be measured that was measured by the radar 180.

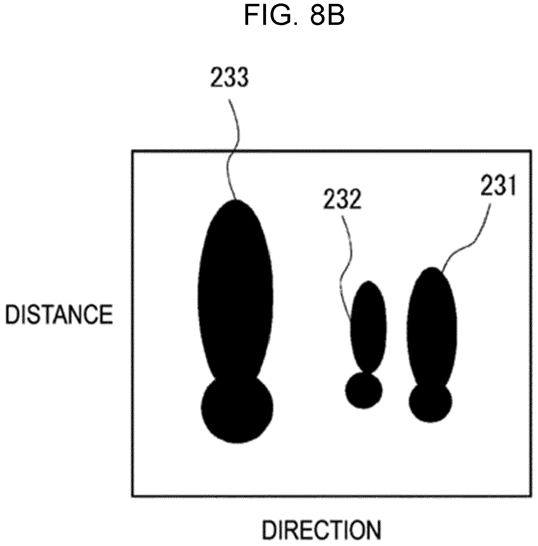

In the present embodiment, in step S306, the transmission/reception unit 111 acquires the feature information indicating the spatial distribution occupied by the persons 31, 32, 33 to be measured that was measured by the radar 180. FIG. 8B shows an example of the feature information of the spatial distributions occupied by the persons 31, 32, 33 to be measured, which is received by the transmission/reception unit 111 from the radar 180 in the present embodiment. As shown in FIG. 8B, a region 231 (size: medium), a region 232 (size: small), and a region 233 (size: large) indicating the spatial distribution according to the body sizes of the persons 31, 32, 33 to be measured, respectively, are output from the radar 180 as position information.

Then, in step S310, the biological information association unit 126 associates the persons 31, 32, 33 to be measured with the biological information on the basis of a degree of similarity between the features of the spatial distributions of the persons 31, 32, 33 to be measured output from the radar 180 and acquired in step S306 and the features of the spatial distributions of the persons 31, 32, 33 to be measured stored in the storage unit 113. For example, the biological information association unit 126 determines that the region 231 is the body temperature distribution of the person 31 to be measured from the feature (size: medium) of the region 231 indicating the spatial distribution, and associates the biological information acquired from the candidate region 51 corresponding to the position of the region 231 with the person 31 to be measured. Similarly, the biological information association unit 126 associates the biological information acquired from the candidate regions 52, 53 with the persons 32, 33 to be measured, respectively.

Therefore, according to the present embodiment, on the basis of the position information of the spatial distribution of each person to be measured acquired by the radar for spatial distribution detection, it is possible to accurately associate the biological information acquired for a plurality of persons to be measured by a biological information acquisition unit using a contactless method, such as a radar, with each person to be measured.

### (Fifth Embodiment)

Next, the biological information processing device according to a fifth embodiment will be described. Note that, in the following description, the same configurations, processing, and the like as those of the embodiments described above are denoted by the same reference signs, and detailed description thereof will be omitted.

In the biological information processing device 100 according to the first embodiment, each of the candidate regions 51, 52, 53 and each of the persons 31, 32, 33 to be measured are associated with each other on the basis of the position information of each person to be measured acquired by optically measuring the outline of each person whose biological information is to be measured by a LiDAR scanner. However, in the biological information processing device 100 according to the fifth embodiment, a voice, a respiratory sound, or the like of each person whose biological information is to be measured is measured by an array microphone, for example, and the candidate regions 51, 52, 53 and the persons 31, 32, 33 to be measured are associated on the basis of feature information indicating a waveform of the sound emitted by each person to be measured and a generation location of the sound. Further, in the present embodiment, before the processing of the flowchart illustrated in FIG. 4 is started, information indicating that the persons whose biological information is to be acquired are the persons 31, 32, 33 to be measured and information related to the feature (e.g., waveform of the voice or the respiratory sound) related to the biological information of each person to be measured that is acquired from the array microphone are stored in advance in the storage unit 113. Alternatively, the user of the biological information processing device 100 may specify this information before the processing of the flowchart is started.

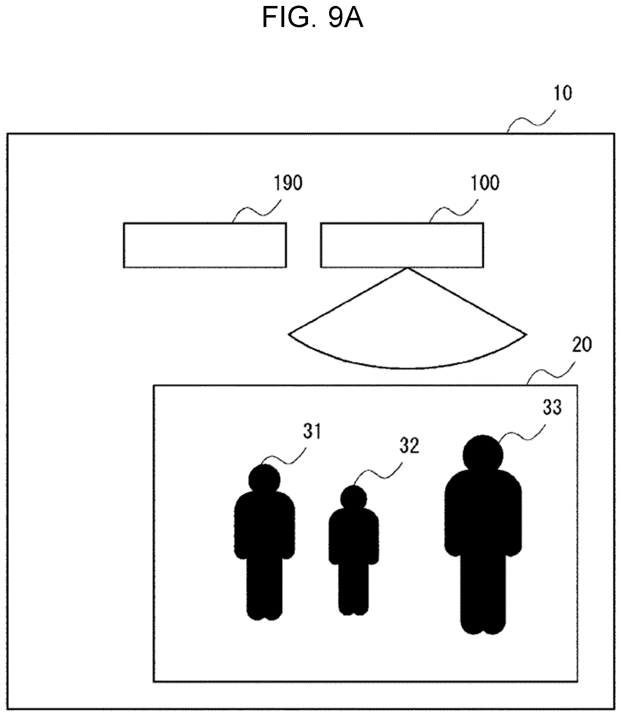

FIG. 9A illustrates a usage example of the biological information processing device 100 according to the present embodiment. As illustrated in FIG. 9A, an array microphone 190 is disposed in the room 10. The array microphone 190 measures a sound emitted by each of the persons 31, 32, 33 to be measured in each direction, and outputs information indicating a waveform of the sound and a generation location of the sound to the biological information processing device 100.

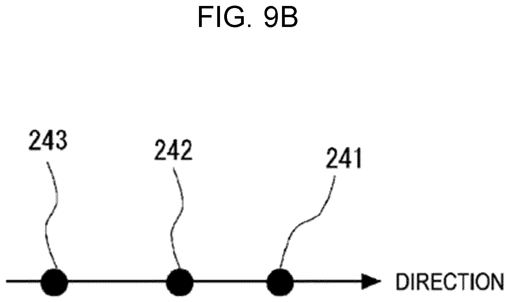

In the present embodiment, in step S306, the transmission/reception unit 111 acquires information indicating the generation locations of the sounds emitted by the persons 31, 32, 33 to be measured that were measured by the array microphone 190. FIG. 9B illustrates an example of the position information indicating the generation locations of the sounds emitted by the persons 31, 32, 33 to be measured received by the transmission/reception unit 111 from the array microphone 190 in the present embodiment. As illustrated in FIG. 9B, directions indicating generation locations 241, 242, 243 of the sounds emitted by the persons 31, 32, 33 to be measured are output from the array microphone 190 as position information.

Then, in step S310, the biological information association unit 126 determines that the generation locations 241, 242, 243 of the sounds are the voices, the respiratory sounds, or the like respectively emitted by the persons 31, 32, 33 to be measured on the basis of a degree of similarity between the waveforms of the sounds emitted by the persons 31, 32, 33 to be measured output from the array microphone 190 and acquired in step S306 and the waveforms of the voices, the respiratory sounds, or the like of the persons 31, 32, 33 to be measured stored in the storage unit 113, and associates the biological information acquired from the candidate regions 51, 52, 53 corresponding to the generation locations 241, 242, 243 of the sounds with the persons 31, 32, 33 to be measured, respectively.

Therefore, according to the present embodiment, on the basis of the position information indicating the generation location of the sound emitted by each person to be measured acquired by an array microphone, it is possible to accurately associate the biological information acquired for a plurality of persons to be measured by a biological information acquisition unit using a contactless method, such as a radar, with each person to be measured.

### (Sixth Embodiment)

Next, the biological information processing device according to a sixth embodiment will be described. Note that, in the following description, the same configurations, processing, and the like as those of the embodiments described above are denoted by the same reference signs, and detailed description thereof will be omitted.

In the biological information processing device 100 according to the first embodiment, each of the candidate regions 51, 52, 53 and each of the persons 31, 32, 33 to be measured are associated with each other on the basis of the position information of each person to be measured acquired by optically measuring the outline of each person whose biological information is to be measured by a LiDAR scanner. However, in the biological information processing device 100 according to the sixth embodiment, each person whose biological information is to be measured wears a radio frequency identification (RFID) tag, and the candidate regions 51, 52, 53 and the persons 31, 32, 33 to be measured are associated with each other on the basis of position information indicating detected positions of the RFID tags. Further, in the present embodiment, before the processing of the flowchart illustrated in FIG. 4 is started, information indicating that the persons whose biological information is to be acquired are the persons 31, 32, 33 to be measured and identification (ID) information of the RFID tag of each person to be measured are stored in advance in the storage unit 113. Alternatively, the user of the biological information processing device 100 may specify this information before the processing of the flowchart is started.

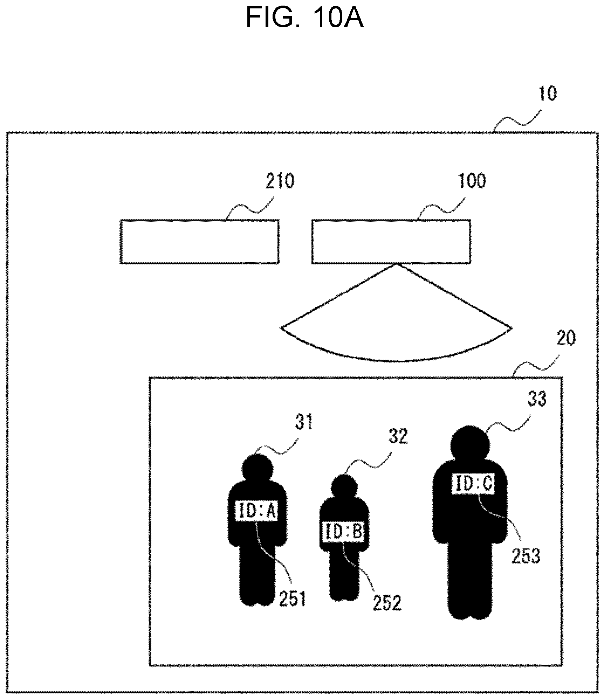

FIG. 10A illustrates a usage example of the biological information processing device 100 according to the present embodiment. As illustrated in FIG. 10A, a detection device 210 for the RFID tags is disposed in the room 10. Further, the persons 31, 32, 33 to be measured wear RFID tags 251, 252, 253 having different ID information ("ID: A," "ID: B," and "ID: C" in the drawing), respectively. The detection device 210 detects the RFID tags 251, 252, 253 and outputs the information of each tag and position information indicating a detected position to the biological information processing device 100. The transmission/reception unit 111 acquires the information of the RFID tag of each person to be measured and the position information indicating the detected position detected by the detection device 210.

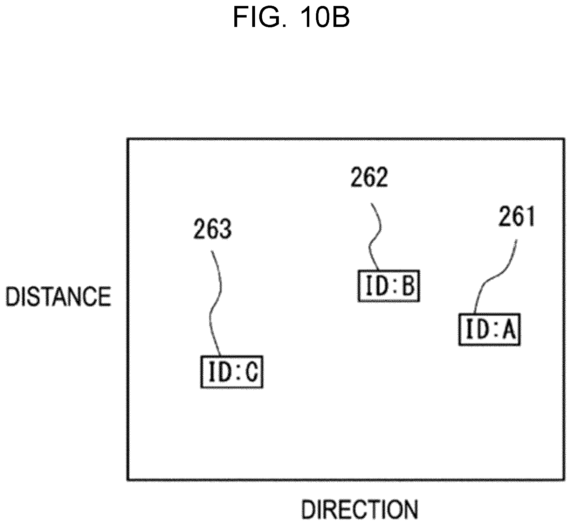

In the present embodiment, in step S306, the transmission/reception unit 111 acquires the information of the RFID tags 251, 252, 253 of the persons 31, 32, 33 to be measured and the position information indicating the detected positions detected by the detection device 210. FIG. 10B illustrates an example of the information of the RFID tags 251, 252, 253 of the persons 31, 32, 33 to be measured and the position information indicating the detected positions, which are received by the transmission/reception unit 111 from the detection device 210 in the present embodiment. As illustrated in FIG. 10B, the ID information of the RFID tags 251, 252, 253 of the persons 31, 32, 33 to be measured and the detected positions 261, 262, 263 are output as position information from the detection device 210.

Then, in step S310, the biological information association unit 126 determines that the persons 31, 32, 33 to be measured are present at the detected positions 261, 262, 263 of the RFID tags 251, 252, 253 on the basis of a comparison between the information of the RFID tags 251, 252, 253 of the persons 31, 32, 33 to be measured acquired in step S306 and the ID information of the RFID tags of the persons 31, 32, 33 to be measured stored in the storage unit 113, and associates the biological information acquired from the candidate regions 51, 52, 53 corresponding to the detected positions 261, 262, 263 with the persons 31, 32, 33 to be measured, respectively.

Therefore, according to the present embodiment, on the basis of the ID information of tags and position information indicating the detected positions acquired by detection of RFID tags worn by persons to be measured, it is possible to accurately associate the biological information acquired for a plurality of persons to be measured by a biological information acquisition unit using a contactless method, such as a radar, with each person to be measured.

### <Other>

The embodiments described above are merely illustrative of configuration examples of the present invention. The present invention is not limited to the specific aspects described above, and various modifications are possible within the scope of the technical idea of the present invention. Modified examples of the embodiments described above will be described below. Note that, in the following description, the same configurations, processing, and the like as those of the embodiments described above are denoted by the same reference signs, and detailed description thereof will be omitted. Further, each of the embodiments described above and the modified examples described below can be implemented in combination as appropriate.

### (First Modified Example)

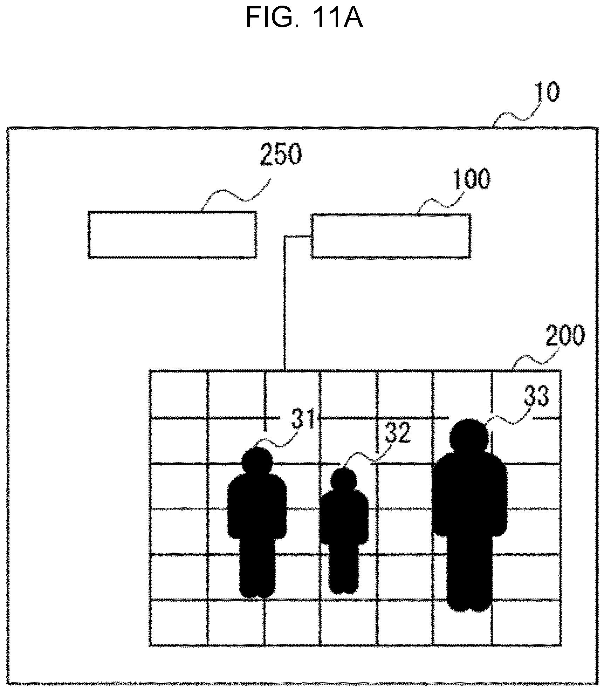

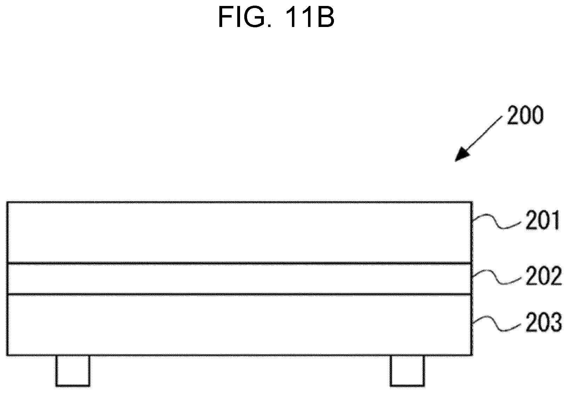

The biological information processing device 100 according to a first modified example will be described below. In this modified example, as illustrated in FIG. 11A, a bed 200 provided with pressure measurement elements is used instead of the bed 20. As illustrated in FIG. 11B, the bed 200 is configured by layering a pressure measurement element group 202 and a mattress 201 on a base portion 203. The pressure measurement element group 202 is configured by pressure measurement elements arranged in a grid on a plane. With this configuration, signals indicating positions of the persons 31, 32, 33 whose biological information is to be measured on the bed 200, positions of the pressure measurement elements, and magnitudes of each pressure are transmitted to the transmission/reception unit 111 of the biological information processing device 100. Accordingly, the reception unit 122 can receive signals indicating the pressure applied to different positions by the plurality of persons to be measured. Further, in the present modified example, a LiDAR scanner 250 is disposed in the room 10. Note that the LiDAR scanner 250 corresponds to the LiDAR scanner 150 of the first embodiment, and thus a detailed description thereof will be omitted here.

Further, in the present embodiment, before the processing of the flowchart illustrated in FIG. 4 is started, information indicating that the persons whose biological information is to be acquired are the persons 31, 32, 33 to be measured and information related to the feature (e.g., feature of the outline of the body of each person to be measured) related to the biological information of each person to be measured acquired from the LiDAR scanner 250 are stored in advance in the storage unit 113. Alternatively, the user of the biological information processing device 100 may specify this information before the processing of the flowchart is started.

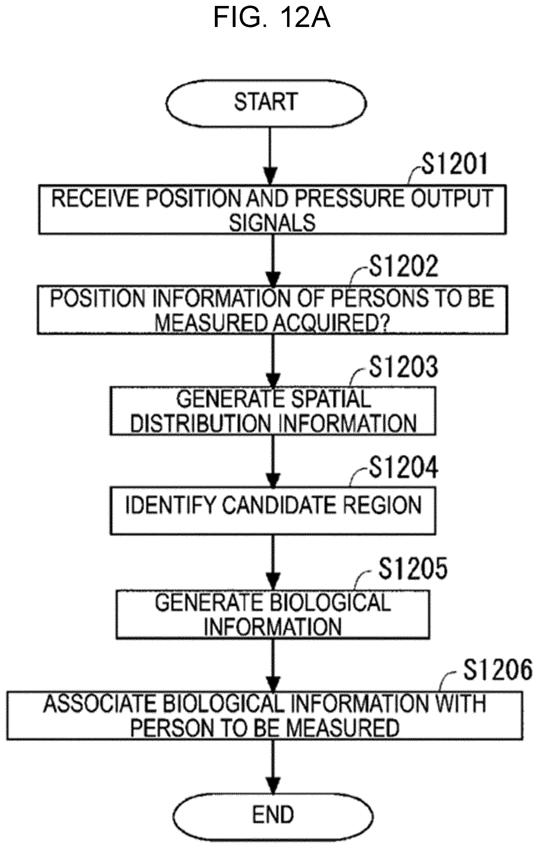

FIG. 12A illustrates an example of a processing flow executed by the control unit 112 in the present modified example. In step S1201, the reception unit 122 receives an output signal of each position and pressure of the pressure measurement elements from the pressure measurement element group 202. Next, in step S1202, the transmission/reception unit 111 acquires the position information of the persons 31, 32, 33 to be measured that was measured by the LiDAR scanner 250.

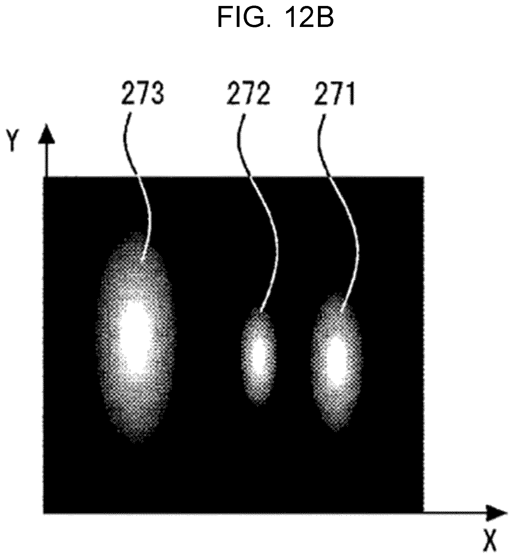

Next, in step S1203, the information generation unit 124 generates information related to the spatial distribution of the pressure indicated by the pressure signals received by the reception unit 122 on the basis of the output signals from the pressure measurement element group 202. Then, in step S1204, the information generation unit 124 specifies candidate regions of the persons to be measured on the basis of the output signals from the pressure measurement element group 202. FIG. 12B shows an example of candidate regions specified by the information generation unit 124 in the present modified example. As shown in FIG. 12B, candidate regions 271, 272, 273 are specified in XY orthogonal coordinates defining a plane on which the pressure measurement element group 202 is disposed. Note that the candidate regions 271, 272, 273 correspond to the persons 31, 32, 33 to be measured, respectively.

Next, in step S1205, the information generation unit 124 generates biological information indicating a respiratory waveform from a temporal change in amplitude or phase for each of the candidate regions 271, 272, 273 specified in step S1204 using the output signals from the pressure measurement element group 202.

Then, in step S310, the biological information association unit 126 associates the persons 31, 32, 33 to be measured with the biological information on the basis of a degree of similarity between the features of the outlines of the bodies of the persons 31, 32, 33 to be measured indicated by the position information of the persons 31, 32, 33 to be measured that were measured by the LiDAR scanner 250 and acquired in step S306 and the features related to the outlines of the bodies of the persons 31, 32, 33 to be measured stored in the storage unit 113. For example, the biological information association unit 126 determines that the point cloud 201 is the outline of the person 31 to be measured from the feature (size: medium) of the outline indicated by the point cloud 201, and associates the biological information acquired from the candidate region 51 corresponding to the position of the point cloud 201 with the person 31 to be measured. Similarly, the biological information association unit 126 associates the biological information acquired from the candidate regions 52, 53 with the persons 32, 33 to be measured, respectively.

According to the present modified example, it is possible to acquire biological information by a contactless method from pressure measurement elements or the like and accurately associate the biological information acquired for a plurality of persons to be measured with each of the persons to be measured.

### (Second Modified Example)

Next, the biological information processing device 100 according to a second modified example will be described. In the embodiments described above, inappropriate situations may arise when identifying the correspondence between the person to be measured and the biological information, such as, for example, a case in which the position or posture of the person whose biological information is to be measured is not appropriate for acquiring the biological information, such as when the person to be measured is bending his/her waist or back, or a case in which the candidate region cannot be appropriately identified because the reflector is not appropriately worn. To deal with this, in the present modified example, it is possible to notify the user of an error when such an inappropriate situation occurs.

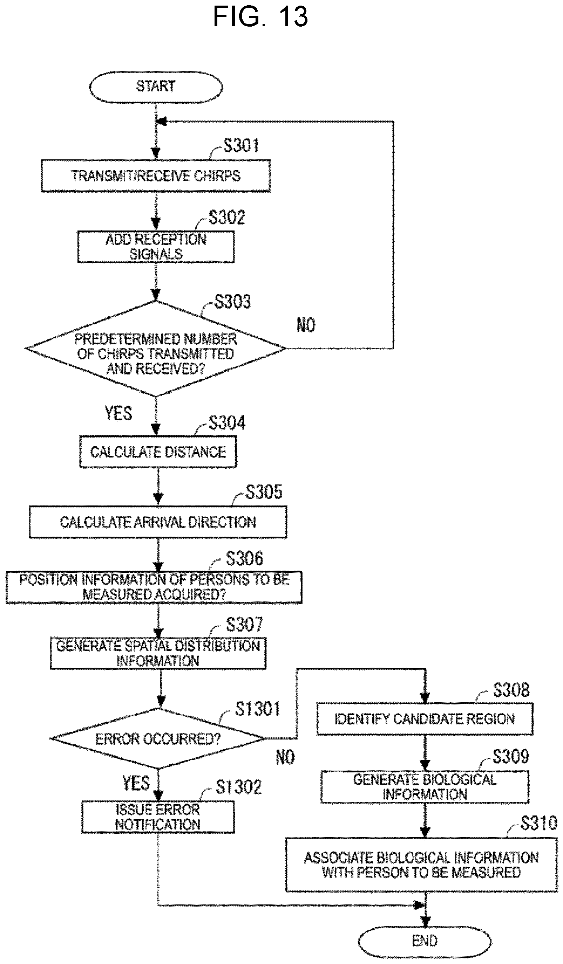

FIG. 13 illustrates an example of a processing flow executed by the control unit 112 in the present modified example. In this processing flow, the processing of steps S301 to S310 is the same as that described above, and thus detailed description thereof will be omitted. In step S1301, on the basis of information related to the spatial distribution generated in step S307, the control unit 112 determines whether there is a possibility that the process of identifying the candidate region of the person to be measured, the process of generating biological information, and the like cannot be normally executed, and determines whether an error has occurred.

For example, in the first embodiment, when the shape of any one of the point groups 201 to 203 acquired from the LiDAR scanner 150 is abnormal, the control unit 112 determines that there is a possibility that the processing described above cannot be normally executed because the posture of the person to be measured is not appropriate, and that an error has occurred. Further, for example, in the second embodiment, when the shape of any one of the regions 211 to 213 of the pressure distribution acquired from the body pressure sensor 160 is abnormal, the determination is made that there is a possibility that the processing described above cannot be normally executed because the posture of the person to be measured is not appropriate, and that an error has occurred. Further, for example, in the fifth embodiment, when the sound emitted by the person to be measured cannot be recognized by the array microphone 190, the determination is made that there is a possibility that the processing described above cannot be normally executed, and that an error has occurred. Note that criteria for determining that the shape of the point group or region, the voice recognition, or the like is abnormal may be determined as appropriate in accordance with the type of biological information to be generated or the like.

In a case where it is determined that an error occurred (S1301 : YES), the control unit 112 advances the processing to step S1302. In a case where it is determined that an error has not occurred (S1302: NO), the control unit 112 advances the processing to step S308. In step S1302, the control unit 112 functions as a notification unit, generates information indicating the determination result of step S1301 and information indicating a solution to resolve the error, and outputs the information from the output unit 114 to, for example, the display device 300, thereby notifying the user of the information. Examples of the information indicating a solution for resolving the error include information urging adjustment of the position of the LiDAR scanner 150, information urging the person to be measured to return to the correct posture, and information urging a voice recognition test of the array microphone 190.

According to the present modified example, it is possible to accurately associate a plurality of persons to be measured with the biological information acquired for each person to be measured by a biological information acquisition unit using a contactless method, such as a radar, while issuing notifications of potential abnormalities that may hinder acquisition of the biological information of the persons to be measured, thus helping to prevent the occurrence of an abnormality.

Furthermore, in the embodiments described above, it is assumed that the transmission/reception unit 111 transmits and receives radio waves to and from the persons whose biological information is to be measured. However, as another modified example, the transmission/reception unit 111 may transmit and receive ultrasonic waves, sound waves, light of a desired wavelength, or the like to and from the persons whose biological information is to be measured. Note that, when ultrasonic waves or sound waves are transmitted and received to and from the persons to be measured, the control unit 112 in the embodiments described above does not execute the process of calculating the distance using the signals transmitted and received by the transmission/reception unit 111, and instead executes a process of calculating the arrival directions of the signals transmitted and received by the transmission/reception unit 111 to associate the person to be measured with the biological information.

Further, as another modified example, the biological information processing device 100 may receive, from a user, an input related to positions (e.g., sleeping areas) of the persons 31, 32, 33 whose biological information is to be measured on the bed 20 as position information indicating spaces occupied by the persons to be measured during measurement, and associate the persons 31, 32, 33 to be measured with the biological information on the basis of the position information received using a correspondence relationship between the candidate regions 251, 252, 253 and the biological information generated and the feature information of the persons 31, 32, 33 to be measured stored in the storage unit 113. Further, a switch or the like, one for each subject, for notifying the biological information processing device 100 of entry of the person to be measured may be installed in the room 10 and the transmission/reception unit 111 may receive information regarding the on/off state of the switch, or the transmission/reception unit 111 may communicate with an information terminal carried by the person to be measured and receive information from the information terminal to identify the candidate region corresponding to the person to be measured on the basis of the received information in the embodiments described above. Furthermore, the biological information processing device 100 may receive information identifying a person to be measured who is present in the room 10 as advance information before measurement as an input from a user or from an external source. Accordingly, the accuracy of the association of the person to be measured with the biological information can be expected to improve.

Further, in the embodiments described above, the signal addition unit 123 performs signal addition by adding the IF signals acquired from the difference between the chirp transmitted by the transmission unit 121 and the signal received by the reception unit 122, and the information generation unit 124 processes the signal obtained by addition to generate the biological information. However, the signal addition by the signal addition unit 123 may be omitted, and the information generation unit 124 may process the IF signal acquired from the difference between the chirp transmitted by the transmission unit 121 and the signal received by the reception unit 122 to generate the biological information.

### (Third Modified Example)

The biological information processing device 100 according to a third modified example will be described below. In the present modified example, as in the first embodiment illustrated in FIG. 1, as an example, a case is assumed in which the biological information processing device 100, the LiDAR scanner 150, and the bed 20 are arranged in the room 10, the plurality of persons 31, 32, 33 whose biological information is to be measured lie on the bed 20, and biological information related to respiration during the sleep of each person to be measured is acquired by the biological information processing device.

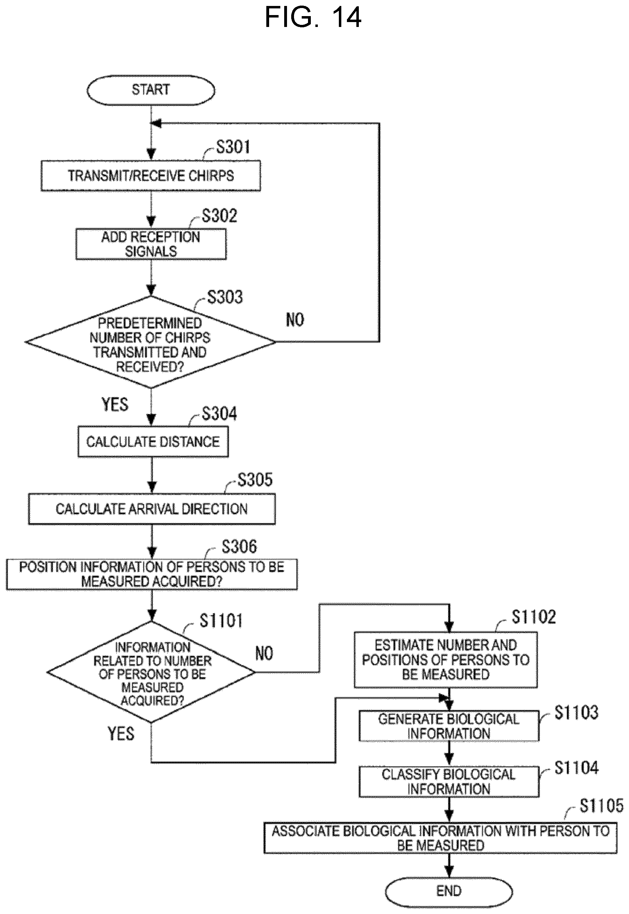

FIG. 14 is a flowchart illustrating an example of a processing flow of the biological information processing device 100. The processing in steps S301 to S306 is the same as that in the first embodiment. More specifically, in step S304, the information generation unit 124 performs Fourier transform on a received signal acquired by the FMCW method and decomposes the received signal into a complex number signal for each distance bin. Further, in step S305, the information generation unit 124 decomposes the received signal into a complex number signal for each direction bin using direction estimation by a phased array radar. Then, the processing in step S304 and step S305 is performed to generate data of the complex number signal (referred to as "Bscope") decomposed into two dimensions of distance x direction. Then, in step S306, as in the first embodiment, the transmission/reception unit 111 acquires the position information of the persons 31, 32, 33 to be measured that was measured by the LiDAR scanner 150.

In step S1101, the control unit 112 determines whether information identifying the number of persons to be measured in the room 10 has been acquired. In the present modified example, the information is acquired by the control unit 112 receiving an input from a user via an input unit (not illustrated) of the biological information processing device 100, or by the transmission/reception unit 111 receiving an input by communicating with an external source. Further, the information may be stored in the storage unit 113 in advance prior to the start of the processing of this flowchart, and the control unit 112 may acquire the information stored in the storage unit 113. The control unit 112 advances the processing to step S1103 in a case in which the information identifying the number of persons to be measured is acquired (S1101: YES), and advances the processing to step S1102 in a case in which the information identifying the number of persons to be measured has not been acquired (S1101: NO).

In step S1102, the information generation unit 124 performs a process of detecting the positions of the persons in the room 10 using the complex number signal data (Bscope) of a plurality of frames, thereby identifying the distance and the direction of the persons to be measured and subject to respiration extraction processing. In the process of detecting the positions of the persons to be measured, the information generation unit 124, as the estimation unit, compares changes in a time difference of the signal for each bin and estimates the number and positions of the persons to be measured by machine learning. Here, the direction bin and/or the distance bin corresponds to an example of the arrival direction and/or the distance of a predetermined unit, but the predetermined unit may be one bin or may be a plurality of bins.

Next, in step S1103, the information generation unit 124 generates biological information indicating a respiratory waveform from a temporal change in amplitude or phase using the signal obtained by addition in step S302.

Next, in step S1104, the information generation unit 124, as the classification unit, classifies the biological information generated in step 1103 on the basis of the information identifying the number of persons to be measured determined to have been acquired in step S1101 or on the basis of the information related to the number and positions of the persons to be measured estimated in step S1102.

Here, an example of the classification of the biological information in the present modified example will be described. Respiratory rates of the three persons 31, 32, 33 to be measured in the room 10 are assumed to be 15 RR/min, 20 RR/min, and 10 RR/min, respectively. At this time, when the number of persons to be measured estimated in step S1101 is estimated to be three, the information generation unit 124 classifies the biological information generated in step 1102 into biological information of three persons on the basis of a difference in respiratory rate. However, in the estimation processing in step S1101, the estimated number of persons to be measured may not match the number of persons to be measured in the room 10 due to detection omission or erroneous detection of the persons to be measured or other factors. In this case, for example, the biological information in which the respiratory rate is to be classified as 15 RR/min is erroneously included in the biological information in which the respiratory rate is to be classified as 10 RR/min or 20 RR/min, or the biological information in which the respiratory rate is to be classified as 20 RR/min is erroneously divided and classified as the biological information of a plurality of persons. As a result, there is a possibility that the persons to be measured and the biological information are not correctly associated with each other.

In view of this, in the present modified example, the control unit 112 serves as an acquisition unit and acquires information identifying the number of persons to be measured in the room 10. In a case in which information identifying the number of persons to be measured is acquired, the information generation unit 124 classifies the biological information using the number of persons indicated by the information instead of the information related to the number and positions of the persons to be measured estimated by the estimation processing in step S1102. Accordingly, it is possible to classify biological information using information having higher accuracy than the information of the number of persons to be measured identified by inference.

Then, in step S1105, the biological information association unit 126 associates the biological information generated by the information generation unit 124 with each person to be measured. Specifically, the biological information association unit 126 associates the persons 31, 32, 33 to be measured with the biological information classified in step S1104 on the basis of a degree of similarity between the features of the outlines of the bodies of the persons 31, 32, 33 to be measured indicated by the position information of the persons 31, 32, 33 to be measured that were measured by the LiDAR scanner 150 and acquired in step S306 and the features related to the outlines of the bodies of the persons 31, 32, 33 to be measured stored in the storage unit 113.

According to the biological information processing device 100 of the present modified example, information related to the number of persons to be measured in the room 10 is acquired, and thus improvements in the accuracy of the association of the biological information with each person to be measured can be expected.

### <Supplementary Note 1>

A biological information processing device including:
a signal reception unit (111) configured to receive a signal related to biological information reflected from at least one person to be measured;
a candidate region identification unit (125) configured to calculate an arrival direction of the signal and/or a distance to the at least one person to be measured from the signal received and identify a candidate region of the at least one person to be measured using the arrival direction and/or the distance calculated;
an information generation unit (124) configured to generate biological information corresponding to the candidate region of the at least one person to be measured from the signal received;
a position information acquisition unit (111) configured to acquire position information of the at least one person to be measured; and
a biological information association unit (126) configured to associate the at least one person to be measured with the biological information generated, on the basis of the position information acquired.

### <Supplementary Note 2>

A biological information processing device including:
a signal reception unit (111) configured to receive signals related to biological information reflected from a plurality of persons;
a candidate region identification unit (125) configured to calculate an arrival direction of each of the signals and/or a distance to at least one person to be measured of the plurality of persons from the signals received and identify a candidate region of the at least one person to be measured using the arrival direction and/or the distance calculated;
an information generation unit (124) configured to generate biological information corresponding to the candidate region of the at least one person to be measured from the signals received;
a position information acquisition unit (111) configured to acquire position information of the at least one person to be measured; and
a biological information association unit (126) configured to associate the at least one person to be measured with the biological information generated, on the basis of the position information acquired.

### <Supplementary Note 3>

A biological information processing device including:
a signal reception unit (111) configured to receive signals indicating pressure applied to different positions by at least one person to be measured;
a candidate region identification unit (125) configured to identify a candidate region of the at least one person to be measured, on the basis of a distribution of the pressure acquired from the signals received;
an information generation unit (124) configured to generate biological information corresponding to the candidate region of the at least one person to be measured from the signals received;
a position information acquisition unit (111) configured to acquire position information of the at least one person to be measured; and
a biological information association unit (126) configured to associate the at least one person to be measured with the biological information generated, on the basis of the position information acquired.

### <Supplementary Note 4>

A biological information processing method executed by a biological information processing device, the method including:
receiving a signal related to biological information reflected from at least one person to be measured (S301);
calculating an arrival direction of the signal and/or a distance to the at least one person to be measured from the signal received and identifying a candidate region of the at least one person to be measured using the arrival direction and the distance calculated (S308);
generating biological information corresponding to the candidate region of the at least one person to be measured from the signal received (S309);
acquiring position information of the at least one person to be measured (S306); and
associating the at least one person to be measured with the biological information generated, on the basis of the position information acquired (S310).

### <Supplementary Note 5>

A biological information processing method executed by a biological information processing device, the method including:
receiving signals related to biological information reflected from a plurality of persons (S301);
calculating an arrival direction of each of the signals and/or a distance to at least one person to be measured of the plurality of persons from the signals received and identifying a candidate region of the at least one person to be measured using the arrival direction and/or the distance calculated (S308);
generating biological information corresponding to the candidate region of the at least one person to be measured from the signals received (S309);
acquiring position information of the at least one person to be measured (S306); and
associating the at least one person to be measured with the biological information generated, on the basis of the position information acquired (S310).

### <Supplementary Note 6>

A biological information processing method executed by a biological information processing device, the method including:
receiving a signal related to biological information reflected from at least one person to be measured (S301);
estimating, by machine learning, a number of the persons to be measured from the signal received for, in terms of an arrival direction of the signal and/or a distance to the at least one person to be measured, each arrival direction and/or distance of a predetermined unit (S1101);
generating biological information of the at least one person to be measured from the signal received (S1102);
classifying the biological information generated for the at least one person to be measured, on the basis of the estimated number of the persons to be measured (S1103);
acquiring position information of the at least one person to be measured (S306); and
associating the at least one person to be measured with the biological information classified, on the basis of the position information acquired (S1104), wherein
in the classification, in a case where information related to the number of the persons to be measured is acquired, the biological information generated for the at least one person to be measured is classified on the basis of the information acquired instead of the estimated number of the persons to be measured.

### <Supplementary Note 7>

A biological information processing method executed by a biological information processing device, the biological information processing method including:
receiving a signal related to biological information reflected from at least one person to be measured (S301);
generating biological information of the at least one person to be measured from the signal received (S1102);
acquiring information related to a number of the persons to be measured (S1103);
classifying the biological information generated for the at least one person to be measured, on the basis of the acquired information related to the number of the persons to be measured (S1103);
acquiring position information of the at least one person to be measured (S306); and
associating the at least one person to be measured with the biological information classified, on the basis of the position information acquired (S1104).

### <Supplementary Note 8>

A biological information processing device comprising:
a signal reception unit (111) configured to receive a signal related to biological information reflected from at least one person to be measured;
an estimation unit (124) configured to estimate, by machine learning, a number of the persons to be measured from the signal received for, in terms of an arrival direction of the signal and/or a distance to the at least one person to be measured, each arrival direction and/or distance of a predetermined unit;
an information generation unit (124) configured to generate biological information of the at least one person to be measured from the signal received;
a classification unit (124) configured to classify the biological information generated for the at least one person to be measured, on the basis of the estimated number of the persons to be measured;
a position information acquisition unit (111) configured to acquire position information of the at least one person to be measured; and
a biological information association unit (126) configured to associate the at least one person to be measured with the biological information classified, on the basis of the position information acquired, wherein
in a case where information related to the number of the persons to be measured is acquired, the classification unit classifies the biological information generated for the at least one person to be measured, on the basis of the information acquired instead of the estimated number of the persons to be measured.

### <Supplementary Note 9>

A biological information processing device including:
a signal reception unit (111) configured to receive a signal related to biological information reflected from at least one person to be measured;
an information generation unit (124) configured to generate biological information of the at least one person to be measured from the signal received;
an acquisition unit (112) configured to acquire information related to a number of the persons to be measured;
a classification unit (124) configured to classify the biological information generated for the at least one person to be measured on the basis of the estimated number of the persons to be measured;
a position information acquisition unit (111) configured to acquire position information of the at least one person to be measured; and
a biological information association unit (126) configured to associate the at least one person to be measured with the biological information classified on the basis of the position information acquired.

### REFERENCE SIGNS LIST

100 biological information processing device, 111 transmission/reception unit, 112 control unit, 124 information generation unit, 125 candidate region identification unit, 126 biological information association unit

## Claims

1. A biological information processing device comprising:
a signal reception unit configured to receive a signal related to biological information reflected from at least one person to be measured;
a candidate region identification unit configured to calculate an arrival direction of the signal and/or a distance to the at least one person to be measured from the signal received and identify a candidate region of the at least one person to be measured using the arrival direction and/or the distance calculated;
an information generation unit configured to generate biological information corresponding to the candidate region of the at least one person to be measured from the signal received;
a position information acquisition unit configured to acquire position information of the at least one person to be measured; and
a biological information association unit configured to associate the at least one person to be measured with the biological information generated, on the basis of the position information acquired.

2. A biological information processing device comprising:
a signal reception unit configured to receive signals related to biological information reflected from a plurality of persons;
a candidate region identification unit configured to calculate an arrival direction of each of the signals and/or a distance to at least one person to be measured of the plurality of persons from the signals received and identify a candidate region of the at least one person to be measured using the arrival direction and/or the distance calculated;
an information generation unit configured to generate biological information corresponding to the candidate region of the at least one person to be measured from the signals received;
a position information acquisition unit configured to acquire position information of the at least one person to be measured; and
a biological information association unit configured to associate the at least one person to be measured with the biological information generated, on the basis of the position information acquired.

3. The biological information processing device according to claim 1 or 2, wherein
the position information of the at least one person to be measured is position information acquired by optically measuring an outline of the at least one person to be measured.

4. The biological information processing device according to claim 1 or 2, wherein
the position information of the at least one person to be measured is position information acquired by measuring a distribution of pressure applied to different positions by the at least one person to be measured.

5. The biological information processing device according to claim 1 or 2, wherein
the position information of the at least one person to be measured is position information acquired by measuring a distribution of body temperature of the at least one person to be measured.

6. The biological information processing device according to claim 1 or 2, wherein
the position information of the at least one person to be measured is position information acquired by measuring a distribution of space occupied by the at least one person to be measured.

7. The biological information processing device according to claim 1 or 2, wherein
the position information of the at least one person to be measured is position information acquired by measuring a sound emitted by the at least one person to be measured and a generation location of the sound.

8. The biological information processing device according to claim 1 or 2, wherein
the position information of the at least one person to be measured is position information indicating a space occupied by the at least one person to be measured during measurement.

9. The biological information processing device according to claim 1 or 2, wherein
the position information of the at least one person to be measured is position information specified by a detected position of a tag worn by the at least one person to be measured and including identification information of the at least one person to be measured.

10. The biological information processing device according to any one of claims 1 to 9, wherein
the biological information association unit associates the at least one person to be measured with the biological information generated, on the basis of information identifying the at least one person to be measured acquired in advance.

11. The biological information processing device according to any one of claims 1 to 10, further comprising:
an output unit configured to output, in a case where a spatial distribution of reflection points of the signal received is abnormal, a notification indicating that the at least one person to be measured and the biological information are not normally associated with each other.

12. A biological information processing device comprising:
a signal reception unit configured to receive signals indicating pressure applied to different positions by at least one person to be measured;
a candidate region identification unit configured to identify a candidate region of the at least one person to be measured, on the basis of a distribution of the pressure acquired from the signals received;
an information generation unit configured to generate biological information corresponding to the candidate region of the at least one person to be measured from the signals received;
a position information acquisition unit configured to acquire position information of the at least one person to be measured; and
a biological information association unit configured to associate the at least one person to be measured with the biological information generated, on the basis of the position information acquired.

13. A biological information processing method executed by a biological information processing device, the method comprising:
receiving a signal related to biological information reflected from at least one person to be measured;
calculating an arrival direction of the signal and/or a distance to the at least one person to be measured from the signal received and identifying a candidate region of the at least one person to be measured using the arrival direction and the distance calculated;
generating biological information corresponding to the candidate region of the at least one person to be measured from the signal received;
acquiring position information of the at least one person to be measured; and
associating the at least one person to be measured with the biological information generated, on the basis of the position information acquired.

14. A biological information processing method executed by a biological information processing device, the method comprising:
receiving signals related to biological information reflected from a plurality of persons;
calculating an arrival direction of each of the signals and/or a distance to at least one person to be measured of the plurality of persons from the signals received and identifying a candidate region of the at least one person to be measured using the arrival direction and/or the distance calculated;
generating biological information corresponding to the candidate region of the at least one person to be measured from the signals received;
acquiring position information of the at least one person to be measured; and
associating the at least one person to be measured with the biological information generated, on the basis of the position information acquired.

15. A biological information processing method executed by a biological information processing device, the method comprising:
receiving a signal related to biological information reflected from at least one person to be measured;
estimating, by machine learning, a number of the persons to be measured from the signal received for, in terms of an arrival direction of the signal and/or a distance to the at least one person to be measured, each arrival direction and/or distance of a predetermined unit;
generating biological information of the at least one person to be measured from the signal received;
classifying the biological information generated for the at least one person to be measured, on the basis of the estimated number of the persons to be measured;
acquiring position information of the at least one person to be measured; and
associating the at least one person to be measured with the biological information classified, on the basis of the position information acquired, wherein
in the classification, in a case where information related to the number of the persons to be measured is acquired, the biological information generated for the at least one person to be measured is classified on the basis of the information acquired instead of the estimated number of the persons to be measured.

16. A biological information processing method executed by a biological information processing device, the method comprising:
receiving a signal related to biological information reflected from at least one person to be measured;
generating biological information of the at least one person to be measured from the signal received;
acquiring information related to a number of the persons to be measured;
classifying the biological information generated for the at least one person to be measured, on the basis of the acquired information related to the number of the persons to be measured;
acquiring position information of the at least one person to be measured; and
associating the at least one person to be measured with the biological information classified, on the basis of the position information acquired.

17. A program for causing a computer to execute each step of the biological information processing method according to any one of claims 13 to 16.

18. A biological information processing device comprising:
a signal reception unit configured to receive a signal related to biological information reflected from at least one person to be measured;
an estimation unit configured to estimate, by machine learning, a number of the persons to be measured from the signal received for, in terms of an arrival direction of the signal and/or a distance to the at least one person to be measured, each arrival direction and/or distance of a predetermined unit;
an information generation unit configured to generate biological information of the at least one person to be measured from the signal received;
a classification unit configured to classify the biological information generated for the at least one person to be measured, on the basis of the estimated number of the persons to be measured;
a position information acquisition unit configured to acquire position information of the at least one person to be measured; and
a biological information association unit configured to associate the at least one person to be measured with the biological information classified, on the basis of the position information acquired, wherein
in a case where information related to the number of the persons to be measured is acquired, the classification unit classifies the biological information generated for the at least one person to be measured, on the basis of the information acquired instead of the estimated number of the persons to be measured.

19. A biological information processing device comprising:
a signal reception unit configured to receive a signal related to biological information reflected from at least one person to be measured;
an information generation unit configured to generate biological information of the at least one person to be measured from the signal received;
an acquisition unit configured to acquire information related to a number of the persons to be measured;
a classification unit configured to classify the biological information generated for the at least one person to be measured, on the basis of the acquired information related to the number of the persons to be measured;
a position information acquisition unit configured to acquire position information of the at least one person to be measured; and
a biological information association unit configured to associate the at least one person to be measured with the biological information classified, on the basis of the position information acquired.
